# EUROPÄISCHE PATENTSCHRIFT

(11) **EP 2 931 258 B1**
(45) Veröffentlichungstag und Bekanntmachung des Hinweises auf die Patenterteilung: **25.09.2019**
(21) Anmeldenummer: 13830175.9
(22) Anmeldetag: 12.12.2013
(51) Int. Cl.: A61K 9/00

(54) **ZUSAMMENSETZUNGEN UND LÖSUNGEN FÜR DIE DARMREINIGUNG**
COMPOSITIONS AND SOLUTIONS FOR COLON CLEANSING
COMPOSITIONS ET SOLUTIONS POUR LE LAVAGE INTESTINAL

(30) Priorität: 14.12.2012 DE 102012024434
(43) Veröffentlichungstag der Anmeldung: 21.10.2015
(73) Patentinhaber: Regalismons S.A., 1840 Luxemburg (LU)
(72) Erfinder: ALBRECHT, Uwe, Willi, 31303 Burgdorf (DE)
(74) Vertreter: Thiel, Christian
(86) Internationale Anmeldenummer: PCT/DE2013/000790
(87) Internationale Veröffentlichungsnummer: WO 2014/090223

(56) Entgegenhaltungen:
- WO-A1-2009/036906
- WO-A2-2006/094737
- WO-A2-2006/134492
- DE-A1-102006 001 199
- US-A1- 2008 038 336
- Anamika Swami: "uses and sources of Calcium sulfate", , 30 December 2011 (2011-12-30), Retrieved from the Internet: URL:https://b2bbusinessnews.wordpress.com/ 2011/12/30/uses-and-sources-of-calcium-sul fate/ [retrieved on 2017-10-02]

## Beschreibung

Die Erfindung betrifft wässrige Lösungen, die wenigstens ein Polysiloxan enthalten, für die orale oder rektale Applikation bei Mensch und Tier und für die Darmreinigung oder zur Prophylaxe oder Therapie bei verschiedenen Verdauungsstörungen und schließlich Verpackungseinheiten aus mehreren Präparaten, die die vorgenannten Zusammensetzungen ergeben.

Polysiloxane sind wichtige Entschäumer, die u.a. in der Medizin, der Lebensmitteltechnologie und anderen technischen Anwendungen zum Einsatz kommen. Sie dienen u.a. als technische Schmierstoffe, als Entschäumer in Farben und Lacken, als Schaumverhüter in Konfitüren, Konserven und Fritierfetten und in der Medizin als Antiflatulanz und Abführmittel.

Ein häufig verwendeter Vertreter dieser Gruppe ist Polydimethylsiloxan (PDMS), ein Silicon der allgemeinen Formel R3Si-0-(R2Si-0-),,SiR3, mit R = Methyl. Die INN-Bezeichnung für PDMS ist Dimeticon. Dimeticon ist farblos und gilt als ungiftig und chemisch weitgehend inert. Es ist als Lebensmittelzusatzstoff und Arzneistoff zugelassen und wird aufgrund seiner entschäumenden Wirkung u.a. als Mittel gegen Gasansammlungen im Magen-Darmtrakt verwendet, wie sie bei verschiedenen Verdauungsstörungen auftreten (Blähungen, Meteorismus). Vor bildgebenden Untersuchungen des Magen-Darm-Trakts wird Polysiloxan gegeben, um Abschattungen durch Gasansammlungen zu vermeiden.

Die Wirkung von Dimeticon als Entschäumer beruht vermutlich darauf, dass die Anwesenheit sowohl polarer wie unpolarer Molekülbereiche eine verbesserte Drainage der Schaumlamellen und damit die Zerstörung des
Schaums bewirken.

An Stelle von Dimeticon wird auch Simeticon verwendet, bei dem es sich um ein Gemisch aus Dimeticon und hochdispersem Siliciumdioxid handelt. Dabei sind üblicherweise 4 - 7 Gew.% SiO₂ in PDMS mit einem Polymerisationsgrad zwischen 200 und 400 enthalten.

Aus der DE 43 41 165 C1 ist die Verwendung von Dimeticon gegen Obstipationen bekannt, insbesondere bei Kindern. Die Polysiloxandosis wurde dabei relativ hoch gewählt. Die Wirkung könnte darauf beruhen, dass Blähungen gelöst und somit Obstipationen durch blähungsbedingte Krämpfe verhindert oder beseitigt werden. Als Mittel gegen Blähungen und Völlegefühl sind sowohl Dimeticon als auch Simeticon u.a. aus der WO 2005/099821 A1 bekannt.

Aus der WO 2009/036906 A1 ist es bekannt, Laxantien mit Entschäumern zu kombinieren, insbesondere mit Dimeticon oder Simeticon, um auf diese Weise Blähungen als Nebenwirkungen der Laxantien vermeiden und Obstipationen besser behandeln zu können. Aus der WO 2004/078182 A1 ist es bekannt, Dimeticon oder Simeticon als Mittel gegen Blähungen mit einem löslichen Saccharid als Abführmittel zu kombinieren.

Die DE 38 07 712 A1 offenbart ein Arzneimittel-Trockenpräparat für die Zubereitung einer laxativ wirkenden Trinklösung. Dabei handelt es sich um ein Granulat mit einer fixen Kombination aus PEG mit einem mittleren Molekulargewicht von wenigstens 4000 Dalton, einem Alkalihydrogencarbonat, Zitronensäure und einer Elektrolytmischung aus Natrium- und Kalium-salzen, die in Anpassung an den Körperhaushalt bilanziert sind. Das Präparat kann 0,1 bis 0,2 % Dimeticon zur Unterdrückung einer eventuellen Schaumbildung bei der Zubereitung der Trinklösung sowie im Verdauungstrakt enthalten und außerdem übliche Zusatzstoffe, wie Süßstoffe, Aromen und Farbstoffe. Die laxativ wirkende Trinklösung ist sowohl als reines Antiobstipationsmittel als auch zur Reinigung des Darms, d.h. als

Darmspüllösung, vor therapeutischen oder diagnostischen Eingriffen geeignet.

In der US 2005/0244368 A1 wird vorgeschlagen, eine elektrolytfreie PEG-Zusammensetzung für die Darmreinigung zu verwenden, insbesondere für Kinder. Darin wird ausgeführt, dass die bekannten elektrolythaltigen PEG-Zusammensetzungen sich gerade für die Vorbereitung einer Colonoskopie nicht als sicher, d.h. mit zu vielen Nebenwirkungen behaftet, erwiesen hätten.

Die US 2008/038336 A1 beschreibt eine feste pharmazeutische Zusammensetzung mit einem Motilitätsregulierer, die Simethicon, Diluent, Calciumsulfat oder Natriumchlorid als Streckmittel sowie PEG als Bindemittel enthält.

Darmspüllösungen, die Polyethylenglykol - INN-Name Macrogol - enthalten, sind seit wenigstens 1984 in USA und Europa in Gebrauch und verdrängen zunehmend die orthograden Darmspülungen mit einfach bilanzierten Elektrolytlösungen und Darmspüllösungen auf Manitolbasis. Die Darmspüllösungen werden zur Entleerung des Darms in Vorbereitung von Eingriffen am oder Untersuchungen im Gastrointestinaltrakt eingesetzt. Die rein salinen Darmspüllösungen waren für den Patienten sehr belastend, weil große Mengen Flüssigkeit aufgenommen werden mussten, weil die salzigen Lösungen die Aufnahme sehr erschwerten und weil die Verträglichkeit eingeschränkt war. Die polyethylenglykolhaltigen Darmspüllösungen brachten einen erheblichen Fortschritt, reinigen den Darm schneller und besser mit geringerer Flüssigkeitsmenge und sind im Allgemeinen gut verträglich. Auf den Zusatz von Laxantien kann weitgehend verzichtet werden. Teilweise werden Elektrolyte, wie Natriumsulfat, Natriumhydrogencarbonat, Natriumchlorid und Kaliumchlorid eingesetzt. Der Patient nimmt üblicherweise zwischen 2 und 6 I einer solchen Darmspüllösung ein. Um beim Spülen Gase aus dem Verdauungstrakt besser abzuführen, kann diesen polyethylenglykolhaltigen Darmspüllösungen Dimeticon oder Simeticon zugesetzt werden. Dies gilt auch und gerade, wenn die Darmspüllösung nicht zur vollständigen Entleerung und Reinigung des Darmes, sondern in geringerer Menge als osmotisches Laxans eingesetzt wird. Dabei wird ausgenutzt, dass Polyethylenglykol reichlich Wasser bindet, einer Verfestigung des Stuhls während der Darmpassage entgegenwirkt und so die Stuhlentleerung erleichtert.

Der Erfindung liegt nun die Aufgabe zugrunde, die Entschäumungswirkung von Polysiloxanen, insbesondere innerhalb von Darmreinigungs- und Antiobstipationsmitteln, weiter zu verbessern und so die Mittel wirkungsvoller und verträglicher zu machen.

Überraschenderweise wurde gefunden, dass der Zusatz von Calciumsulfat zu Zusammensetzungen, die Polysiloxan als Entschäumer enthalten, eine signifikante und nicht zu erwartende Verbesserung der Entschäumungswirkung bewirken.

Entsprechend betrifft die Erfindung eine wässrige Lösung zur oralen oder rektalen Verabreichung bei Mensch oder Tier zum Zwecke einer Darmreinigung oder für die Prophylaxe oder Therapie bei Verdauungsstörungen, Obstipation, Flatulenz, Meteorismus, Bauchkrämpfen oder Borborygmus, dadurch gekennzeichnet, dass sie Polyethylenglycol, wenigstens ein Polysiloxan und Calciumsulfat enthält und dass sie in Bezug auf das Calciumsulfat praktisch gesättigt ist, wobei i) das Löslichkeitsprodukt von Calciumsulfat in der Lösung um nicht mehr als 10 % unterschritten wird, oder ii) dass sie diesbezüglich übersättigt ist oder iii) dass sie als Suspension oder kolloidale Lösung mit feindispersem Calciumsulfat vorliegt.

Das zu diesem Zweck eingesetzte Calciumsulfat ist dabei vorzugsweise das Dihydrat, also CaSO₄ • 2 H₂O (Gips). Alternativ ist auch der Einsatz des Hemihydrats CaSO₄ • 1/2 H₂O möglich. Die im Weiteren angegebenen Mengenangaben beziehen sich immer auf das Dihydrat. Bei Verwendung anderer Calciumsulfatmodifikationen bzw. des Hemihydrats ist auf entsprechenden Calciumsulfat-Gehalt umzurechnen.

Das Calciumsulfat wird als feinkristallines Pulver eingesetzt, wie es im Handel erhältlich ist.

Bevorzugt werden der oder die Polysiloxan-Entschäumer und Calciumsulfat in einem Gewichtsverhältnis von 1:1 bis 1:100 in der Zusammensetzung eingesetzt. Dies bedeutet, dass das Calciumsulfat wenigstens in gleicher Menge oder im Überschuss vorhanden ist.

Es ist besonders bevorzugt, wenn das Polysiloxan Dimeticon oder Simeticon ist.

Erfindungsgemäß liegt die Zusammensetzung flüssig, d.h. als Flüssigmischung, kolloidale Lösung oder Suspension vor und wird vorzugsweise innerhalb einer Kapsel, in Form einer Tropflösung, in einem Sirup, in einem Saft oder dergleichen angeboten. Die Zusammensetzung enthält Wasser, wobei sich die Polysiloxane praktisch nicht und CaSO₄ sehr wenig in Wasser lösen. Um die Homogenität der Zusammensetzung zu verbessern, können Glykol und/oder Glycerin enthalten sein. Polysiloxan einerseits und Calciumsulfat andererseits sind in den Zusammensetzungen in fixer Dosiskombination vorhanden (fixed-dose combination).

Es ist bekannt, dass Calciumionen alleine bereits entschäumend wirken können. Hierfür muss es sich jedoch um sehr wässrige (verdünnte) Systeme handeln, wie sie zunächst im Gastrointestinaltrakt nicht vorliegen. Die Calciumionen sind auch in der Lage, Fette auszufällen, die sonst zur Schaumstabilisierung dienen könnten. Dies ist aus der Fett- und Tensid-Chemie bekannt.

Bei der Erfindung scheint nach derzeitiger Erkenntnis wesentlich zu sein, dass das Calciumsulfat innerhalb einer flüssigen Zusammensetzung, d.h. einer Lösung, in kolloidaler Form, feindispers suspendiert, an der Löslichkeitsgrenze oder in übersättigter Lösung vorliegt. Dies wird unten im Zusammenhang mit Lösungen nach der Erfindung noch näher erläutert.

In Weiterbildung der Erfindung enthält die Zusammensetzung zusätzlich
wenigstens einen weiteren Wirkstoff, insbesondere wenigstens ein Laxans. Dafür geeignete Laxantien können vom Fachmann aus üblichen Laxantien ausgewählt werden. Diese umfassen unter anderem:
- Füll- und Quellstoffe, wie pflanzliche Fasern, Inulin, verschiedene Saaten;
- Gleitmittel, wie Glycerin, Paraffin;
- Motilitätssteigende Mittel, wie Rhabarber, Rizinus, Sennoiside (Sennesblätter)
- Osmotisch wirkende Mittel, die Wasser binden und so einer Stuhlentwässerung entgegenwirken, wie Bittersalz, Glaubersalz, Zucker-alkohole (Sorbitol, Mannitol, Xylitol) und Polyethylenglykol (INN-Name-Macrogol).

Die oben beschriebene wässrige Lösung nach der Erfindung kann wie dargestellt unmittelbar oral oder rektal gegeben werden, sodass das Polysiloxan seine Wirkung in Verbindung mit dem Calciumsulfat unmittelbar im Körper, und zwar im Gastrointestinaltrakt einschließlich Rektum, entfaltet.

Der Begriff "Lösung" umfasst neben echten Lösungen der Inhalts-stoffe im Lösungsmittel auch übersättigte Lösungen, Lösungen mit Bodensatz, Suspensionen und insbesondere kolloidale Lösungen mit fein-dispersem Calciumsulfat, ggf. mit weiteren nicht echt gelösten, sondern nur aufgeschwemmten (suspendierten) oder eine mulgierten Inhaltsstoffen.

Die erfindungsgemäße wässrige Lösung ist vorzugsweise eine Darmspüllösung, wie sie zur Vorbereitung operativer Eingriffe im Gastrointestinaltrakt oder zu endoskopischen Untersuchungen sowie Ultraschalluntersuchungen verwendet wird. Die Lösung kann ebenfalls als osmotisches Antiobstipationsmittel, d.h. zur Förderung der Stuhlentleerung ohne vollständige Darmreinigung vorgesehen sein.

Erfindungsgemäße Lösungen zur oralen oder rektalen Verabreichung bei Mensch oder Tier zum Zwecke einer Darmreinigung oder für die Prophylaxe oder Therapie bei Verdauungsstörungen, Obstipation, Flatulenz, Meteorismus usw. enthalten Polyetylenglykol, wenigstens ein Polysiloxan und Calciumsulfat in wässriger Lösung, wobei der Begriff "wässrige Lösung"
unechte Lösungen umfasst, nämlich kolloidale Lösungen, die Feststoffe oder Flüssigkeiten in kolloidaler Form enthalten, Suspensionen und Emulsionen. Beispielsweise sind die Polysiloxane, die in Wasser praktisch nicht löslich sind, sowie in Wasser schwer lösliche oder unlösliche Gleitmittel und dergleichen als Flüssigkeiten kolloidal gelöst oder emulgiert. Das feste Calciumsulfat, welches in Wasser ebenfalls nur sehr schwer löslich ist (Löslichkeit ca. 2g pro Liter bei 20°C) kann ebenfalls kolloidal gelöst oder suspendiert werden. Das Calciumsulfat wird vorzugsweise an seiner Löslichkeitsgrenze verwendet. Es kann daher bei der Herstellung der erfindungsgemäßen Lösung - insbesondere, wenn dies bei erhöhter Temperatur geschieht - auch zeitweilig gelöst oder in übersättigter Lösung vorliegen. Wenn ein zusätzlicher Bodensatz an Calciumsulfat vorhanden ist, ist zugleich die Maximalkonzentration an Ionen in der erfindungsgemäßen Lösung vorhanden. Das Löslichkeitsprodukt von Calciumsulfat gibt hierfür die Obergrenze an.

Die erfindungsgemäße Lösung besitzt die verschiedensten Anwendungsmöglichkeiten. Primär handelt es sich um eine Darmspüllösung. Die Darmspüllösung kann bei Patienten, die vor eine Koloskopie eine orthograde Darmlavage benötigen, durchgeführt werden, bei Patienten, bei denen vor einer Operation, das heißt einem chirurgischen Eingriff, eine Darmspülung erforderlich ist, bei Patienten mit infektiöser Besiedelung des Darm mit Erregern, die durch Spülen saniert werden soll (z. B. Salmonellenenteritis), zur Vorbereitung von Patienten zur Radiologie, z. B. vor der Doppelkontrastbarium-Darstellung, bei Fach-Doppler-Untersuchungen, zur Vorbereitung einer Notfalldiagnostik, insbesondere bei rektalen Blutungen und bei akuten Vergiftungszuständen als gastroindestinale Dekontaminationstechnik.

Die Darmspüllösung kann von der Zusammensetzung her unverändert, jedoch ggf. in geringerer Gesamtflüssigkeitsmenge, auch zur Behandlung von Obstipationen angewendet werden, und zwar bei chronischen oder akuten Obstipationen bis hin zu sehr starken Obstipationen, wie der Koprostase. Auch bei bestimmten Erkrankungen ist für eine erleichterte Defäkation zu sorgen, so dass die erfindungsgemäßen Lösungen angezeigt sind, zum Beispiel auch bei postoperativen Obstipationen und bei durch Medikamente verursachten Obstipationen (z. B. Opiatobstipationen). Eine weitere Indikation für die erfindungsgemäße Darmspüllösung ist die Prophylaxe und Therapie der portokavalen Enzephalopathie.

Unter anderem auch für die vorgenannten Zwecke haben sich seit länger Zeit polyethylenglykolhaltige Darmspüllösungen als besonders vorteilhaft erwiesen. Ein Vorteil der Anwendung derartiger Zubereitungen liegt in der Reduktion der Vorbereitungszeit von früher in der Regel drei Tagen auf ein bis zwei Tage mit einem Minimum von 4-5 Stunden. Dabei sind vom Patienten zwischen zwei und sechs Litern der fertigen Darmspüllösung einzunehmen. Mit dieser Spülmenge von mehreren Litern wird der Darm vollständig entleert und gereinigt, was mit sich bringt, dass die Lösung dem Körper wichtige Stoffe entziehen könnte. Dies ist zu vermeiden. Um die Aufnahme von Wasser und Elektrolyten aus der Darmwand in das Darmlumen zu verhindern, werden Darmspüllösungen häufig Elektrolyte zugesetzt. Höhere Elektrolytverluste stellen erhebliche Risiken dar. Unter hohen Elektrolytverlusten wäre die Anwendung einer Darmspüllösung nicht mehr sicher und könnte schlimmstenfalls lebensbedrohlich sein. Es wird daher eine Netto-Nullwanderung von Wasser und Elektrolyten angestrebt. Hierfür versucht man Isoosmolarität zwischen der zubereiteten Lösung und der intra- bzw. extrazellulären Flüssigkeit der Darmwand herzustellen.

Eine sehr vorteilhafte Darmspüllösung, die neben Polyethylenglykol bereits Dimeticon oder Simeticon als Polysiloxan-Entschäumer enthält, ist aus der DE 10 2006 001 199 A1 bekannt.

Die aus der DE 10 2006 001 199 A1 bekannten Darmspüllösungen werden im Rahmen der hier vorliegenden Erfindung mit Calciumsulfat als entschäumungsverstärkendem Mittel ergänzt.

Ein wesentlicher Aspekt der Erfindung ist, dass die Lösung, wie oben beschrieben, in Bezug auf das Calciumsulfat praktisch gesättigt ist, wobei das Löslichkeitsprodukt von Calciumsulfat um nicht mehr als 10% unterschritten wird oder dass die Lösung bezüglich Calciumsulfat übersättigt ist oder dass sie als Suspension oder kolloidale Lösung mit feindispersem Calciumsulfat vorliegt. Ein Bodensatz von Calciumsulfat ist möglich, der Bodensatz kann vor der Verabreichung durch Rühren aufgeschlemmt und so mitverabreicht werden.

Bei allen Lösungen nach der Erfindung, also Darmspüllösungen oder Lösungen als Antiobstipationsmittel, sollte das Polyethylenglycol ein Molekulargewicht von zwischen 2000 Da und 6000 Da besitzen, vorzugsweise zwischen 3000 Da und 4000 Da und besonders bevorzugt bei 3350 Da oder 4000 Da. Makrogole mit diesen Molekulargewichten sind für entsprechende Zwecke bewährt. Gemäß einem Aspekt der Erfindung können die Lösungen zusätzlich wenigstens einen Elektrolyten ausgewählt aus der Gruppe physiologisch verträglicher Kalium-, Natrium- und Magnesiumsalze enthalten, ausgenommen mit solchen Anionen, die mit Calcium in Wasser schwerlösliche Salze bilden und insbesondere ausgenommen in Form von Sulfaten, Carbonaten und Oxalaten. Elektrolyte, die zur Herstellung einer Isoosmolarität im Darm bereitgestellt werden, werden daher vorzugsweise als Chloride und/oder Hydrogencarbonate eingesetzt. Da sich nur sehr wenig Calciumsulfat in Wasser löst, wird die Elektrolytwirkung durch das Calciumsulfat nur wenig verändert, bzw. die gegebene Veränderung kann leicht berücksichtigt werden. Die Verwendung von Bittersalz und Glaubersalz wird also hier vermieden, was bereits des unangenehm bitteren Geschmacks wegen angezeigt ist. Es hatte sich herausgestellt, dass die sulfathaltigen Mittel eine schlechte Complience besitzen und häufig zu Erbrechen führen.

An Stelle von Bittersalz und Glaubersalz können jedoch andere Laxantien zugefügt werden. Bevorzugt sind dabei Gleitmittel, neutrale Quellmittel oder auch Antiflatulantien, wie insbesondere solche auf pflanzlicher Basis. Beispiele sind Kümmelöl, Fenchelöl, Pfefferminzöl, Anisöl, Kamillenextrakt, Myrrhe. Die erfindungsgemäßen Darmspüllösungen können weitere Wirkstoffe enthalten, die jedoch keinen Einfluss auf den Elektrolythaushalt nehmen sollten und nicht krampfauslösend oder blähend wirken dürfen. Als weitere Wirkstoffe können insbesondere Detoxikationsmittel, Antiemetika oder Propulsiva enthalten sein.

Gemäß einem alternativen Aspekt der Erfindung werden Darmspüllösungen bereitgestellt, die nicht mit Elektrolyten ergänzt sind. Auch diese Zusammensetzungen haben, wie beispielsweise in der US 2005/0244368 A1 beschrieben Ihre Anwendungsgebiete.

Die Lösungen nach der Erfindung können außerdem Zusatz- und Hilfsstoffe enthalten, wie Aromen, Süßstoffe, Farbstoffe, Stabilisatoren, Konservierungsstoffe, Vitamine und dergleichen.

Im Folgenden wird die Erfindung anhand von Beispielen näher erläutert, die ausschließlich dazu dienen sollen, die Erfindung besser zu illustrieren. Der Fachmann kann anhand der vorstehenden Beschreibung ohne Weiteres weitere Beispiele auffinden. Die Erfindung ist nicht auf die genannten Beispiele beschränkt.

### BEISPIELE:

### Beschreibung der Inhaltsstoffe

Polyethylenglykole (Macrogole) sind kommerziell erhältlich. Solche mit Molekulargewichten von 2000 Da bis 6000 Da sind im Rahmen der Erfindung geeignet. Besonders geeignet scheinen Polyethylenglykole mit Molekulargewichten zwischen 3000 Da und 4000 Da. In den Beispielen wurde ein Polyethylenglykol mit dem Molekulargewicht 3350 Da (PEG 3350) verwendet. Die Polyethylenglykole sind Feststoffe, sie werden pulverförmig eingesetzt.

Die Elektrolyte sind einfache Salze und werden als feinkristalline Feststoffe in möglichst reiner Form eingesetzt. Sie sind alle kommerziell erhältlich. In den Beispielen wird ein Elektrolytgemisch aus Natrium- und Kaliumsalzen verwendet, wobei im Einzelnen Natriumchlorid und Kaliumchlorid sowie Natriumhydrogencarbonat verwendet werden.

Polysiloxane: Es wurden sowohl Dimeticon als auch Simeticon untersucht. Der INN-Name Dimeticon steht für α-(Trimethylsilyl)-ω-methylpoly (oxydimethylsilylen)), INCI: Dimethicone. Flüssigkeit, praktisch unlöslich in Wasser, mischbar mit Glykol und Glycerol.

Simeticon ist ein Gemisch aus Dimeticon und Siliziumdioxid. Das Siliziumdioxid ist dabei in feindisperser, vorzugsweise nanodisperser Form (pyrogene Kieselsäure). Der Siliziumdioxidgehalt kann variieren. INCI: Simethicone. Suspension aus Siliziumdioxid in Dimeticon, viskosflüssig und grauweiß opaleszierend.

Das Calciumsulfat wird in den Beispielen als Dihydrat in feinkristalliner Form eingesetzt. Es ist kommerziell erhältlich. Calciumsulfat ist als Lebensmittelzusatz zugelassen und kann in dieser Qualität oder als pharmazeutischer Rohstoff in entsprechender Qualität hier verwendet werden.

Das Löslichkeitsprodukt von Calciumsulfat beträgt 2,3 x 10⁻⁴ (mol²/L²). Dieses Löslichkeitsprodukt K_{L} = [Ca²⁺] [SO₄²⁺] würde durch zusätzlich aus anderen Quellen in der Lösung vorhandene Calciumionen und Sulfationen beeinflusst. In den Beispielen werden die Lösungen daher mit entmineralisiertem Wasser angesetzt.

### Beispiel 1

In 500ml entmineralisiertem Wasser werden gelöst

| | |
|---|---|
| 52,5 g | Polyethylenglycol 3350 |
| 0,715g | Natriumhydrogencarbonat |
| 1,400g | Natriumchlorid |
| 0,185g | Kaliumchlorid |

In diese Lösungen wurden unter schnellem Rühren 0,25g Simeticon in 0,5g Glycerol getropft. Zusätzlich wurde 1g feinkristallines Calciumsulfatdihydrat eingerührt.

Die Lösung ist frisch, das heißt, innerhalb von 20 Minuten ab Herstellung zu verwenden.

### Beispiel 2

Zu Beispiel 1 wurden 0,5g Saccharin-Natrium als Süßungsmittel und 0,5g eines Fruchtaromas gegeben.

### Beispiel 3

1g feinkristallines trockenes Calciumsulfat wird mit 0,35g Simeticon imprägniert. Die Mischung kann in einer Vibrations-Mühle erfolgen. Das mit Simeticon imprägnierte Calciumsulfat wird unter starken Rühren in 500ml entmineralisiertes Wasser eingerührt, das zuvor mit 60g PEG 3350 versetzt worden war.

Die Mischung wird zehn Minuten weitergerührt und innerhalb von 20 Minuten verwendet.

### Beispiel 4

1g feinkristallines trockenes Calciumsulfat wird mit 0,25 g Dimeticon imprägniert. Die Mischung kann in einer Vibrations-Mühle erfolgen. Das mit Dimeticon imprägnierte Calciumsulfat wird unter starken Rühren in 500ml entmineralisiertes Wasser eingerührt, das zuvor mit 60g PEG 3350 versetzt worden war.

Die Mischung wird fünf Minuten gerührt, weitergerührt und innerhalb von 20 Minuten verwendet.

### TESTS

Es wurden in vitro-Untersuchungen zur Schaumbildung in verschiedenen Spüllösungen durchgeführt. Als Basis wurden kommerziell erhältliche Macrogol-Spüllösungen verwendet. Die unveränderten Lösungen wurden bezüglich ihres Schaumbildungsverhaltens bei einem standardisierten Schütteltest (Bartsch-Test) mit solchen Lösungen verglichen, die 1.) zusätzlich Simeticon, 2.) zusätzlich CaSO₄, aber kein Simeticon, 3.) sowohl CaSO₄ als auch Simeticon enthielten. Die Ergebnisse werden nachfolgend berichtet.

Die Schaumbildung wurde durch den Kurzzeit-Bartsch-Test ermittelt. Hierbei werden 5 ml der Test-Lösung in ein graduiertes Reagenzglas mit einem Durchmesser von 15 mm und einem Füllungsvolumen von 20 ml gegeben. Unmittelbar darauf wird in einem Zeitraum von 7 s 10mal standardisiert geschüttelt und das Volumen des dabei entstandenen Schaums gemessen. Zudem wird die Zeit bis zum ersten Sichtbarwerden der Lösung bestimmt oder das stabile Restvolumen nach 5 min. ermittelt.

Folgende Lavage Lösungen wurden in vitro untersucht (Stammlösungen):
**A: Endofalk Classic,** Wirkstoffe: Kaliumchlorid, Natriumchlorid, Natriumhydrogencarbonat, Macrogol 3350,
**B: Medicoforum Cleansing,** Wirkstoffe: Kaliumchlorid, Natriumchlorid, Natriumhydrogencarbonat, Macrogol 3350, Mengenzusammensetzung wie in Beispiel 1,
**C: Klean-Prep,** Wirkstoffe: Kaliumchlorid, Natriumchlorid, Natriumsulfat, Natriumhydrogencarbonat, Macrogol 3350, Aspartam, Vanille-Arom astoff,
**D: Moviprep Orange,** Wirkstoffe: Kaliumchlorid, natriumchlorid, Natriumsulfat, Ascorbinsäure, Natriumascorbat, Macrogol 3350.

Die Stammlösungen A - D wurden entsprechend der im Beipackzettel angegebenen Anleitungen bei Zimmertemperatur (22 °C) in den vorgeschriebenen Konzentrationen zusammengestellt. Es wurden jeweils 500 ml Stammlösung angesetzt.

Von diesen Stammlösungen A - D wurden jeweils 5 ml für den Bartsch-Test verwendet.

Ist der Restschaum nach 5 min ausreichend stabil, so werden in einer zweiten Serie 500 ml der Test-Lösung 0,2 ml Simeticon (entsprechen etwa 5 mg Simeticon) hinzugegeben und der Kurzzeit-Bartsch-Test wiederholt. Jede Untersuchung wird 10 mal durchgeführt, und die Mittelwerte und Standardabweichungen werden berechnet. Die statistischen Analysen erfolgten mittels Student-t Test. Signifikante Unterschiede ergaben sich bei einem p < 0,05.

**Tabelle 1: Schaumvolumen und Auflösungszeit ohne und mit Simeticon in den Standardlösugnen (n= 10)**

| | | **Schaumvolumen (ml)** | **Auflösungszeit (sec)** |
|---|---|---|---|
| | | Mittelwerte und Standardabweichungen | |
| **Lösung A** | ohne Simeticon | 1,30 +/- 0,13 | 88 +/- 11,6 |
| | mit Simeticon | 0,20 +/- 0,60 | 4 +/- 3,0 |
| **Lösung B** | ohne Simeticon | 1,20 +/- 0,90 | 187 +/- 79 |
| | mit Simeticon | 0,10 +/- 0,15 | 11 +/- 1,50 |
| **Lösung C** | ohne Simeticon | 2,30 +/- 0,40 | 147 +/- 30,0 |
| | mit Simeticon | 0,10 +/- 0,10 | 7 +/- 0,90 |
| **Lösung** | **D** ohne Simeticon | 1,20 +/- 0,20 | 70 +/- 60 |
| | mit Simeticon | 0,10 +/- 0,03 | 6 +/-2,00 |

### Ergebnisse für den Vergleich Stammlösung mit und ohne Simeticon

Nach dem Bartsch-Test zeigten alle Lösungen A - D eine feinblasige Kugelschaumbildung von etwa 30 % des Volumens der Testlösungen, die für etwa 120 s anhielt. Den meisten Schaum erzeugte die Test-Lösung C mit 2,34 ml und einer Zeit bis zum Sichtbarwerden der Lösung von 147 s. (Tabelle 1). Stabiler war jedoch der Schaum der Lösung B mit einer Auflösungszeit von 187 s. Die reinen Macrogol-Stammlösungen A - D zeigen demnach alle hohe Schaumbildungsneigung. Unter Zugabe von 0,2 ml (5 mg) Simeticon wurde in allen Lösungen A - D eine Schaumbildung weitgehend verhindert. Der Schaum war grobblasig und löste sich schnell auf. Die Schaumvolumina lagen im Schnitt bei 0,13 ml und die Schaum-Auflösungszeit bei 7 s. (Tabelle 1). Alle Unterschiede waren hochsignifikant (p < 0,0001).

### Wirkung von Calciumsulfat auf die Stammlösungen mit und ohne Simeticon.

### Allgemeine Beeinflussung der Spülflüssigkeit

1 g Calciumsulfat auf 500 ml Spülflüssigkeit verändert den Geschmack, dieser wird kalkiger und stumpfer. Calciumsulfat muss sehr gut eingerührt werden, und ein Teil fällt nach etwa 30 min in Ruhe wieder aus.

Nachfolgend sind die Testergebnisse für Stammlösung B wiedergegeben, deren Schaum im Vergleichstest am stabilsten war.

**Tabelle 2:**

| | **Schaumvolumen (ml) n=10** | **Auflösungszeit (sec) (n=10)** |
|---|---|---|
| (1) Stammlösung B ohne Zusätze | 1,27 +/- 0,27 | 125 +/- 48 |
| (2) Stammlösung B mit CaSO₄ | 0,84 +/- 0,13 | 52,4 +/- 30 |
| (3) Stammlösung B mit Simeticon und CaSO₄ | 0,00 | 0,00 |
| Signifikanz | P < 0,0002 | P < 0,0001 |

1 g CaSO₄ und 0,2 ml Simeticon auf 500 ml Stammlösung Testvolumen 5 ml.
Mittelwerte aus 10 Versuchen, n = 10.

### Ergebnisse

Bereits Lösung (2) - nur Stammlösung mit CaSO₄ bildet signifikant weniger Schaum, der zudem signifikant weniger stabil ist. Die Wirkung ist jedoch deutlich geringer als die von Simeticon (siehe Tabelle 1). Vollständig überzeugen können letzlich nur die Ergebnisse für Lösung (3). Dort wurden sowohl CaSO₄ als auch Simeticon zum Entschäumen verwendet. Es entstand kein messbarer Schaum.

### Diskussion

Die Stammspüllösungen bilden zu 30 % ihres Eigenvolumens feinblasigen Kugelschaum, der im Zeitverlauf eine relative Stabilität aufweist. Es wurden für die Untersuchungen nur kleine Volumina der Lösungen verwendet. Eine Hochrechnung auf die normalerweise verwendeten Volumina von 2000-3000 ml würde im Vorbereitungsverlauf von etwa 16 Stunden ein beträchtliches Schaumvolumen von 600 - 1000 ml ergeben. Dadurch können durchaus Blähungen und abdominelle Beschwerden mit Krämpfen bei einem Teil der Patienten erklärt werden
Die Zugabe von Simeticon verhindert die Schaumbildung bereits weitgehend.

Die Lösung mit Calciumsulfat und Simeticon bildete keinen messbaren Schaum. Der Geschmack der Lösung war gegenüber einer entsprechend zubereiteten Lösung ohne Calciumsulfat nur unwesentlich verändert. Die neuen Lösungen sollten daher von den Patienten gut akzeptiert werden können. Die einschlägigen Nebenwirkungen - verursacht durch Schaumbildung - wie Völlegefühl, Blähungen, Übelkeit werden minimiert.

## Patentansprüche

1. Wässrige Lösung zur oralen oder rektalen Verabreichung bei Mensch oder Tier zum Zwecke einer Darmreinigung oder für die Prophylaxe oder Therapie bei Verdauungsstörungen, Obstipation, Flatulenz, Meteorismus, Bauchkrämpfen oder Borborygmus, **dadurch gekennzeichnet, dass** sie Polyethylenglycol, wenigstens ein Polysiloxan und Calciumsulfat enthält und dass sie in Bezug auf das Calciumsulfat praktisch gesättigt ist, wobei i) das Löslichkeitsprodukt von Calciumsulfat in der Lösung um nicht mehr als 10 % unterschritten wird, oder ii) dass sie diesbezüglich übersättigt ist oder iii) dass sie als Suspension oder kolloidale Lösung mit feindispersem Calciumsulfat vorliegt.

2. Wässrige Lösung nach Anspruch 1, **dadurch gekennzeichnet, dass** das Polyethylenglycol ein Molekulargewicht zwischen 2000 Dalton und 6000 Dalton, vorzugsweise 3000 Dalton bis 4000 Dalton, besitzt.

3. Wässrige Lösung nach Anspruch 1 oder 2, **dadurch gekennzeichnet, dass** sie wenigstens einen Elektrolyten, ausgewählt aus der Gruppe physiologisch verträglicher Kalium-, Natrium- und Magnesiumsalze enthält, ausgenommen mit solchen Anionen, die mit Calcium in Wasser schwerlösliche Salze bilden, wie insbesondere ausgenommen Sulfate, Carbonate und Oxalate, und vorzugsweise als Chloride und Hydrogencarbonate.

4. Wässrige Lösung nach einem der Ansprüche 1 bis 3, **dadurch gekennzeichnet, dass** wenigstens ein weiterer Wirkstoff, insbesondere ausgewählt aus der Gruppe Laxantien, Gleitmittel, Antiflatulantien, Detoxikationsmittel, Antiemetika, Propulsiva enthalten ist.

5. Wässrige Lösung nach einem der Ansprüche 1 bis 4, **dadurch gekennzeichnet, dass** Zusatz- und Hilfsstoffe, wie Aromen, Süßstoffe, Farbstoffe, Stabilisatoren, Konservierungsstoffe und/oder Vitamine enthalten sind.

6. Wässrige Lösung nach einem der Ansprüche 1 bis 5, **dadurch gekennzeichnet, dass** Polysiloxan-Entschäumer und Calciumsulfat in einem Gewichtsverhältnis von 1:1 bis 1:100 enthalten sind.

7. Wässrige Lösung nach einem der Ansprüche 1 bis 6, **dadurch gekennzeichnet, dass** das Polysiloxan Dimeticon oder Simeticon ist.

## Claims

1. Aqueous solution for oral or rectal administration in humans or animals for the purpose of bowel cleansing or for prophylaxis or therapy in the case of digestive disorders, constipation, flatulence, meteorism, stomach cramps or borborygmus, **characterized in that** it contains polyethylene glycol, at least one polysiloxane and calcium sulfate and **in that** it is virtually saturated with respect to the calcium sulfate, wherein i) the solubility product of calcium sulfate in the solution is fallen short of by not more than 10%, or ii) **in that** it is oversaturated in this respect or iii) **in that** it is present as a suspension or colloidal solution with finely dispersed calcium sulfate.

2. Aqueous solution according to Claim 1, **characterized in that** the polyethylene glycol has a molecular weight between 2000 daltons and 6000 daltons, preferably 3000 daltons to 4000 daltons.

3. Aqueous solution according to Claim 1 or 2, **characterized in that** it contains at least one electrolyte selected from the group of physiologically compatible potassium, sodium and magnesium salts, except with those anions which form poorly soluble salts with calcium in water, such as, in particular, except sulfates, carbonates and oxalates, and preferably as chlorides and hydrogencarbonates.

4. Aqueous solution according to any of Claims 1 to 3, **characterized in that** at least one further active ingredient is present, especially selected from the group consisting of laxatives, lubricants, antiflatulents, detoxification agents, antiemetics, propulsives.

5. Aqueous solution according to any of Claims 1 to 4, **characterized in that** additives and excipients are present, such as flavourings, sweeteners, colourings, stabilizers, preservatives and/or vitamins.

6. Aqueous solution according to any of Claims 1 to 5, **characterized in that** polysiloxane antifoam and calcium sulfate are present in a weight ratio of from 1:1 to 1:100.

7. Aqueous solution according to any of Claims 1 to 6, **characterized in that** the polysiloxane is dimethicone or simethicone.

## Revendications

1. Solution aqueuse destinée à l'administration orale ou rectale chez l'humain ou l'animal dans le but d'un lavage intestinal ou pour la prophylaxie ou le traitement dans des troubles digestifs, la constipation, la flatulence, le météorisme, les crampes abdominales ou les bruits intestinaux, **caractérisée en ce qu'**elle contient du polyéthylèneglycol, au moins un polysiloxane et du sulfate de calcium et **en ce qu'**elle est pratiquement saturée en ce qui concerne le sulfate de calcium, i) le produit de solubilité du sulfate de calcium dans la solution restant au-dessous de 10 %, ou ii) **en ce qu'**elle est sursaturée à cet égard, ou iii) en ce qu'elle est présente sus forme de suspension ou de solution colloïdale avec du sulfate de calcium finement dispersé.

2. Solution aqueuse selon la revendication 1, **caractérisée en ce que** le polyéthylèneglycol a une masse moléculaire comprise entre 2 000 daltons et 6 000 daltons, de préférence de 3 000 daltons à 4 000 daltons.

3. Solution aqueuse selon la revendication 1 ou 2, **caractérisée en ce qu'**elle contient au moins un électrolyte, choisi dans le groupe des sels de potassium, sodium et magnésium physiologiquement acceptables, hormis avec les anions qui forment avec le calcium des sels difficilement solubles dans l'eau, comme en particulier hormis le sulfates, carbonates et oxalates, et de préférence sous forme de chlorures et d'hydrogénocarbonates.

4. Solution aqueuse selon l'une quelconque des revendications 1 à 3, **caractérisée en ce qu'**est contenue au moins une autre substance active, en particulier choisie dans le groupe des laxatifs, lubrifiants, antiflatulents, détoxifiants, antiémétiques, propulsifs.

5. Solution aqueuse selon l'une quelconque des revendications 1 à 4, **caractérisée en ce que** sont contenus des additifs et adjuvants, tels que des aromes, édulcorants, colorants, stabilisants, conservateurs et/ou vitamines.

6. Solution aqueuse selon l'une quelconque des revendications 1 à 5, **caractérisée en ce que** des antimousses polysiloxane et du sulfate de calcium sont contenus en un rapport pondéral de 1:1 à 1:100.

7. Solution aqueuse selon l'une quelconque des revendications 1 à 6, **caractérisée en ce que** le polysiloxane est la diméticone ou la siméticone.
